# NEUE EUROPÄISCHE PATENTSCHRIFT

(11) **EP 1 445 310 B2**
(45) Veröffentlichungstag und Bekanntmachung des Hinweises auf die Entscheidung über den Einspruch: **29.06.2016**
(45) Hinweis auf die Patenterteilung: 17.05.2006
(21) Anmeldenummer: 04001958.0
(22) Anmeldetag: 29.01.2004
(51) Int. Cl.: C12N 1/14, C12N 1/16, C12N 1/21, C12N 15/52, C12P 13/12

(54) **Verfahren zur fermentativen Herstellung von L-Methionin**
Process for the fermentative production of L-methionine
Procédé de préparation fermentative de L-méthionine

(30) Priorität: 06.02.2003 DE 10305774
(43) Veröffentlichungstag der Anmeldung: 11.08.2004
(73) Patentinhaber: Consortium für elektrochemische Industrie GmbH, 81379 München (DE)
(72) Erfinder: Maier, Thomas, Dr., 85221 Dachau (DE); Winterhalter, Christoph, Dr., 82343 Pöcking (DE); Pfeiffer, Kerstin, 81373 München (DE)
(74) Vertreter: Potten, Holger

(56) Entgegenhaltungen:
- EP-A- 0 885 962
- PATENT ABSTRACTS OF JAPAN Bd. 2000, Nr. 08, 6. Oktober 2000 (2000-10-06) & JP 2000 139471 A (AJINOMOTO CO INC), 23. Mai 2000 (2000-05-23)
- BURLAND V ET AL: "ANALYSIS OF THE ESCHERICHIA COLI GENOME VI: DNA SEQUENCE OF THE REGION FROM 92.8 THROUGH 100 MINUTES" NUCLEIC ACIDS RESEARCH, OXFORD UNIVERSITY PRESS, SURREY, GB, Bd. 23, Nr. 12, 25. Juni 1995 (1995-06-25), Seiten 2105-2119, XP000612159 ISSN: 0305-1048
- BLATTNER ET AL: "Reference No.: A64720" SCIENCE, AMERICAN ASSOCIATION FOR THE ADVANCEMENT OF SCIENCE,, US, Bd. 277, 5. September 1997 (1997-09-05), Seiten 1453-1462, XP002108437 ISSN: 0036-8075
- HAMILTON ET AL: "NEW METHOD FOR GENERATING DELETIONS AND GENE REPLACEMENT IN ESCHERICHIA COLI" JOURNAL OF BACTERIOLOGY, WASHINGTON, DC, US, Bd. 171, Nr. 9, September 1989 (1989-09), Seiten 4617-4622, XP002119417 ISSN: 0021-9193
- MAKRIDES: "Strategies for Achieving High-Level Expression of Genes in Escherichia coli" MICROBIOLOGICAL REVIEWS, AMERICAN SOCIETY FOR MICROBIOLOGY, WASHINGTON, DC, US, Bd. 60, Nr. 3, 1. September 1996 (1996-09-01), Seiten 512-538, XP002095235 ISSN: 0146-0749

### SEQUENCE LISTING

<110> Consortium fuer elektrochemische Industrie GmbH
<120> Verfahren zur fermentativen Herstellung von L-Methionin
<130> yjeH
<140>
   <141>
<160> 10
<170> PatentIn Ver. 2.0
<210> 1
   <211> 1652
   <212> DNA
   <213> Escherichia coli
<220>
   <221> CDS
   <222> (141)..(1394)
<400> 1
   agcacaaaat cgggtgaaaa ccctgattca cctcacattt catcgcaatt tcttcatcgc 60
   cgtcataagc gaatctgatt gtgctaccat cgaaaatcta cgcagttgcc caaaatttgg 120
   gcgcaatcgg acatcaaccc atg agt gga ctc aaa caa gaa ctg ggg ctg gcc 173
                        Met Ser Gly Leu Lys Gln Glu Leu Gly Leu Ala
                          1               5                  10
   cag ggc att ggc ctg cta tcg acg tca tta tta ggc act ggc gtg ttt   221
   Gln Gly Ile Gly Leu Leu Ser Thr Ser Leu Leu Gly Thr Gly Val Phe
                15                  20                  25
   gcc gtt cct gcg tta gct gcg ctg gta gcg ggc aat aac agc ctg tgg   269
   Ala Val Pro Ala Leu Ala Ala Leu Val Ala Gly Asn Asn Ser Leu Trp
            30                  35                  40
   gcg tgg ccc gtt ttg att atc tta gtg ttc ccg att gcg att gtg ttt   317
   Ala Trp Pro Val Leu Ile Ile Leu Val Phe Pro Ile Ala Ile Val Phe
        45                  50                  55
   gcg att ctg ggt cgc cac tat ccc agc gca ggc ggc gtc gcg cac ttc   365
   Ala Ile Leu Gly Arg His Tyr Pro Ser Ala Gly Gly Val Ala His Phe
    60                  65                  70                  75
   gtc ggt atg gcg ttt ggt tcg cgg ctt gag cga gtc acc ggc tgg ctg   413
   Val Gly Met Ala Phe Gly Ser Arg Leu Glu Arg Val Thr Gly Trp Leu
                    80                  85                  90
   ttt tta tcg gtc att ccc gtg ggt ttg cct gcc gca cta caa att gcc   461
   Phe Leu Ser Val Ile Pro Val Gly Leu Pro Ala Ala Leu Gln Ile Ala
                95                 100                 105
   gcc ggg ttc ggc cag gcg atg ttt ggc tgg cat agc tgg caa ctg ttg   509
   Ala Gly Phe Gly Gln Ala Met Phe Gly Trp His Ser Trp Gln Leu Leu
           110                 115                 120
   ttg gca gaa ctc ggt acg ctg gcg ctg gtg tgg tat atc ggt act cgc    557
   Leu Ala Glu Leu Gly Thr Leu Ala Leu Val Trp Tyr Ile Gly Thr Arg
       125                 130                 135
   ggt gcc agt tcc agt gct aat cta caa acc gtt att gcc gga ctt atc    605
   Gly Ala Ser Ser Ser Ala Asn Leu Gln Thr Val Ile Ala Gly Leu Ile
   140                 145                 150                 155
   gtc gcg ctg att gtc gct atc tgg tgg gcg ggc gat atc aaa cct gcg    653
   Val Ala Leu Ile Val Ala Ile Trp Trp Ala Gly Asp Ile Lys Pro Ala
                   160                 165                 170
   aat atc ccc ttt ccg gca cct ggt aat atc gaa ctt acc ggg tta ttt    701
   Asn Ile Pro Phe Pro Ala Pro Gly Asn Ile Glu Leu Thr Gly Leu Phe
               175                 180                 185
   gct gcg tta tca gtg atg ttc tgg tgt ttt gtc ggt ctg gag gca ttt    749
   Ala Ala Leu Ser Val Met Phe Trp Cys Phe Val Gly Leu Glu Ala Phe
           190                 195                 200
   gcc cat ctc gcc tcg gaa ttt aaa aat cca gag cgt gat ttt cct cgt    797
   Ala His Leu Ala Ser Glu Phe Lys Asn Pro Glu Arg Asp Phe Pro Arg
       205                 210                 215
   gct ttg atg att ggt ctg ctg ctg gca gga tta gtc tac tgg ggc tgt    845
   Ala Leu Met Ile Gly Leu Leu Leu Ala Gly Leu Val Tyr Trp Gly Cys
   220                 225                 230                 235
   acg gta gtc gtc tta cac ttc gac gcc tat ggt gaa aaa atg gcg gcg    893
   Thr Val Val Val Leu His Phe Asp Ala Tyr Gly Glu Lys Met Ala Ala
                   240                 245                 250
   gca gca tcg ctt cca aaa att gta gtg cag ttg ttc ggt gta gga gcg    941
   Ala Ala Ser Leu Pro Lys Ile Val Val Gln Leu Phe Gly Val Gly Ala
               255                 260                 265
   tta tgg att gcc tgc gtg att ggc tat ctg gcc tgc ttt gcc agt ctc    989
   Leu Trp Ile Ala Cys Val Ile Gly Tyr Leu Ala Cys Phe Ala Ser Leu
           270                 275                 280
   aac att tat ata cag agc ttc gcc cgc ctg gtc tgg tcg cag gcg caa    1037
   Asn Ile Tyr Ile Gln Ser Phe Ala Arg Leu Val Trp Ser Gln Ala Gln
       285                 290                 295
   cat aat cct gac cac tac ctg gca cgc ctc tct tct cgc cat atc ccg    1085
   His Asn Pro Asp His Tyr Leu Ala Arg Leu Ser Ser Arg His Ile Pro
   300                 305                 310                 315
   aat aat gcc ctc aat gcg gtg ctc ggc tgc tgt gtg gtg agc act ttg    1133
   Asn Asn Ala Leu Asn Ala Val Leu Gly Cys Cys Val Val Ser Thr Leu
                   320                 325                 330
   gtg att cat gct tta gag atc aat ctg gac gct ctt att att tat gcc    1181
   Val Ile His Ala Leu Glu Ile Asn Leu Asp Ala Leu Ile Ile Tyr Ala
               335                 340                 345
   aat ggc atc ttt att atg att tat ctg tta tgc atg ctg gca ggc tgt    1229
   Asn Gly Ile Phe Ile Met Ile Tyr Leu Leu Cys Met Leu Ala Gly Cys
           350                 355                 360
   aaa tta ttg caa gga cgt tat cga cta ctg gcg gtg gtt ggc ggg ctg 1277
   Lys Leu Leu Gln Gly Arg Tyr Arg Leu Leu Ala Val Val Gly Gly Leu
   365 370 375
   tta tgc gtt ctg tta ctg gca atg gtc ggc tgg aaa agt ctc tat gcg 1325
   Leu Cys Val Leu Leu Leu Ala Met Val Gly Trp Lys Ser Leu Tyr Ala
   380 385 390 395
   ctg atc atg ctg gcg ggg tta tgg ctg ttg ctg cca aaa cga aaa acg 1373
   Leu Ile Met Leu Ala Gly Leu Trp Leu Leu Leu Pro Lys Arg Lys Thr
   400 405 410
   ccg gaa aat ggc ata acc aca taatccggcg tttcgacatt aatcctggcg 1424
   Pro Glu Asn Gly Ile Thr Thr
   415
   atcgtcttta tgatcaaggc ggtcgcgctc atcatccttt cgctggtact caccatcaaa 1484
   agtattaccg ccaccggtcc cggcgctaaa accgccgcca ggcatgcgag aaaagcgcaa 1544
   atgcggcatc aacttcactg tcagatgctt ttgcaccggc ggcaataaaa gtagaagacc 1604
   gaggaagtcg gtaaaaaagc ccggtaataa aagcagcaaa ccagcaat 1652
<210> 2
   <211> 418
   <212> PRT
   <213> Escherichia coli
<400> 2
   Met Ser Gly Leu Lys Gln Glu Leu Gly Leu Ala Gln Gly Ile Gly Leu
   1 5 10 15
   Leu Ser Thr Ser Leu Leu Gly Thr Gly Val Phe Ala Val Pro Ala Leu
   20 25 30
   Ala Ala Leu Val Ala Gly Asn Asn Ser Leu Trp Ala Trp Pro Val Leu
   35 40 45
   Ile Ile Leu Val Phe Pro Ile Ala Ile Val Phe Ala Ile Leu Gly Arg
   50 55 60
   His Tyr Pro Ser Ala Gly Gly Val Ala His Phe Val Gly Met Ala Phe
   65 70 75 80
   Gly Ser Arg Leu Glu Arg Val Thr Gly Trp Leu Phe Leu Ser Val Ile
   85 90 95
   Pro Val Gly Leu Pro Ala Ala Leu Gln Ile Ala Ala Gly Phe Gly Gln
   100 105 110
   Ala Met Phe Gly Trp His Ser Trp Gln Leu Leu Leu Ala Glu Leu Gly
   115 120 125
   Thr Leu Ala Leu Val Trp Tyr Ile Gly Thr Arg Gly Ala Ser Ser Ser
   130 135 140
   Ala Asn Leu Gln Thr Val Ile Ala Gly Leu Ile Val Ala Leu Ile Val
   145 150 155 160
   Ala Ile Trp Trp Ala Gly Asp Ile Lys Pro Ala Asn Ile Pro Phe Pro
   165 170 175
   Ala Pro Gly Asn Ile Glu Leu Thr Gly Leu Phe Ala Ala Leu Ser Val
   180 185 190
   Met Phe Trp Cys Phe Val Gly Leu Glu Ala Phe Ala His Leu Ala Ser
   195 200 205
   Glu Phe Lys Asn Pro Glu Arg Asp Phe Pro Arg Ala Leu Met Ile Gly
   210 215 220
   Leu Leu Leu Ala Gly Leu Val Tyr Trp Gly Cys Thr Val Val Val Leu
   225 230 235 240
   His Phe Asp Ala Tyr Gly Glu Lys Met Ala Ala Ala Ala Ser Leu Pro
   245 250 255
   Lys Ile Val Val Gln Leu Phe Gly Val Gly Ala Leu Trp Ile Ala Cys
   260 265 270
   Val Ile Gly Tyr Leu Ala Cys Phe Ala Ser Leu Asn Ile Tyr Ile Gln
   275 280 285
   Ser Phe Ala Arg Leu Val Trp Ser Gln Ala Gln His Asn Pro Asp His
   290 295 300
   Tyr Leu Ala Arg Leu Ser Ser Arg His Ile Pro Asn Asn Ala Leu Asn
   305 310 315 320
   Ala Val Leu Gly Cys Cys Val Val Ser Thr Leu Val Ile His Ala Leu
   325 330 335
   Glu Ile Asn Leu Asp Ala Leu Ile Ile Tyr Ala Asn Gly Ile Phe Ile
   340 345 350
   Met Ile Tyr Leu Leu Cys Met Leu Ala Gly Cys Lys Leu Leu Gln Gly
   355 360 365
   Arg Tyr Arg Leu Leu Ala Val Val Gly Gly Leu Leu Cys Val Leu Leu
   370 375 380
   Leu Ala Met Val Gly Trp Lys Ser Leu Tyr Ala Leu Ile Met Leu Ala
   385 390 395 400
   Gly Leu Trp Leu Leu Leu Pro Lys Arg Lys Thr Pro Glu Asn Gly Ile
   405 410 415
   Thr Thr
<210> 3
   <211> 33
   <212> DNA
   <213> Artificial Sequence
<220>
   <223> Primer for PCR
<400> 3
   **gtcgacgcgt gaggcgagtc agtcgcgtaa tgc 33**
<210> 4
   <211> 43
   <212> DNA
   <213> Artificial Sequence
<220>
   <223> Primer for PCR
<400> 4
   **gaccttaatt aagatctcat atattccacc agctatttgt tag 43**
<210> 5
   <211> 18
   <212> DNA
   <213> Artificial Sequence
<220>
   <223> Primer for PCR
<400> 5
   **attgctggtt tgctgctt 18**
<210> 6
   <211> 18
   <212> DNA
   <213> Artificial Sequence
<220>
   <223> Primer for PCR
<400> 6
   **agcacaaaat cgggtgaa 18**
<210> 7
   <211> 27
   <212> DNA
   <213> Artificial Sequence
<220>
   <223> Primer for PCR
<400> 7
   **cgcccatggc tccttttagt cattctt 27**
<210> 8
   <211> 27
   <212> DNA
   <213> Artificial Sequence
<220>
   <223> Primer for PCR
<400> 8
   **cgcgagctca gtactattaa tccagcg 27**
<210> 9
   <211> 37
   <212> DNA
   <213> Artificial Sequence
<220>
   <223> Primer for PCR
<400> 9
   **gatcgcggcc gctgcaacgc ggcatcatta aattcga 37**
<210> 10
   <211> 36
   <212> DNA
   <213> Artificial Sequence
<220>
   <223> Primer for PCR
<400> 10
   **gatcgcggcc gcagtttcaa ccagttaatc aactgg 36**

## Beschreibung

Die Erfindung betrifft ein Verfahren zur Herstellung von L-Methionin mittels Fermentation.

Die Aminosäure Methionin spielt eine herausragende Rolle in der Tierernährung. Methionin gehört zu den essentiellen Aminosäuren, die im Stoffwechsel der Wirbeltiere nicht durch Biosynthese hergestellt werden können. Demzufolge muß bei der Tierzucht sichergestellt werden, dass Methionin in ausreichenden Mengen mit der Nahrung aufgenommen wird. Da Methionin aber in klassischen Futtermittelpflanzen (wie Soja oder Getreide) insbesondere für Schweine und Geflügel häufig in zu geringen Mengen vorhanden ist, um eine optimale Tierernährung zu gewährleisten, ist es vorteilhaft, Methionin als Zusatzstoff dem Ticrfutter beizumischen. Die hohe Bedeutung von Methionin für die Tierernährung ist auch darauf zurückzuführen, dass Methionin neben L-Cystein (bzw. L-Cystin) die entscheidende Schwefelquelle im Stoffwechsel darstellt. Der tierische Stoffwechsel ist zwar in der Lage Methionin in Cystein zu überführen aber nicht vice versa.

Im Stand der Technik wird Methionin im Maßstab > 100 000 Jahrestonnen durch chemische Synthese hergestellt. Dabei wird zunächst Acrolein und Methylmercaptan zu 3-Methylthiopropionaldehyd umgesetzt, der wiederum mit Cyanid, Ammoniak und Kohlenmonoxid zum Hydantoin führt. Dieses kann letztendlich zum Razemat, einem äquimolaren Gemisch der beiden Stereoisomeren D- bzw. L-Methionin hydrolysiert werden. Da die biologisch aktive Form des Moleküls ausschließlich die L-Form repräsentiert, muss die im Futter enthaltene D-Form im Stoffwechsel erst durch Des- und Transaminierung in die aktive L-Form überführt werden.

Zwar sind Methoden bekannt, die eine Herstellung von enantiomerenreinem L-Methionin durch Razematspaltung odermittels Hydantoinasen erlauben. Aufgrund der hohen Kosten haben diese Methoden in der Futtermittelindustrie aber bisher keinen Einzug gefunden.

Im klaren Gegensatz zu Methionin wird die Herstellung der meisten anderen natürlichen, proteinogenen Aminosäuren heutzutage vorwiegend durch Fermentation von Mikroorganismen bewerkstelligt. Dabei wird ausgenützt, dass Mikroorganismen über entsprechende Biosynthesewege zur Synthese der natürlichen Aminosäuren verfügen. Zudem erreichen viele Fermentationsverfahren mit billigen Edukten wie Glucose und Mineralsalzen sehr günstige Herstellkosten und liefern zudem die biologisch aktive L-Form der jeweiligen Aminosäure.

Biosynthesewege von Aminosäuren unterliegen Jedoch in Wildtyp-Stämmen einer strengen metabolischen Kontrolle, die gewährleistec, dass die Aminosäuren nur zum Eigenbedarf der Zelle hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch gesteigerte Produktionsleistung für die Herstellung der gewünschten Aminosäure aufweisen.

Solche Aminosäure-überproduzierende Mikroorganismen können durch klassische Mutations-/Selektionsverfahren und/oder durch moderne, gezielte, rekombinante Techniken ("metabolic engineering") erzeugt werden. Bei letzterem werden zunächst Gene oder Allele identifiziert, die durch ihre Veränderung, Aktivierung oder Inaktivierung eine Aminosäure-Überproduktion bewirken. Diese Gene/Allele werden dann durch molekularbiologische Techniken in einen Mikroorganismenstamm eingebracht oder inaktiviert, so dass eine optimale Überproduktion erzielt wird. Häufig führt jedoch erst die Kombination mehrerer, verschiedener Maßnahmen zu einer wirklich effizienten Produktion.

Die Biosynthese von L-Methionin in Mikroorganismen ist sehr komplex. Der Aminosäure-Körper des Moleküls leitet sich vom L-Aspartat ab, das über Aspartylsemialdehyd/Aspartyl-Phosphat zu L-Homoserin umgesetzt wird. An diesem Molekül wird dann in drei enzymatischen Schritten (über O-Succinylhomoserin und Cystathionin) die Hydroxylgruppe gegen eine Thiolgruppe ausgetauscht, wobei diese aus einem Cystein-Molekül mobilisiert wird und Homocystein entsteht. Im letzen Schritt der Biosynthese wird schließlich durch Methylierung der Thiolgruppe L-Methionin gebildet. Die Methylgruppe leitet sich aus dem Serin-Stoffwechsel ab.

Formal gesehen wird Methionin im mikrobiellen Stoffwechsel also seinerseits aus den Aminosäuren Aspartat, Serin und Cystein synthetisiert und benötigt damit im Vergleich zu anderen Aminosäuren eine hohe Komplexität der Biosynthese. Neben dem Hauptstrang der Synthese (Aspartat - Homoserin - Homocystein) muss auch die Cystein-Biosynthese und damit die komplexe Fixierung von anorganischem Schwefel sowie der C1-Stoffwechsel optimal abgestimmt werden.

Aus diesen Gründen ist die fermentative Herstellung von L-Methionin in der Vergangenheit nicht sehr intensiv bearbeitet worden. In den letzten Jahren sind jedoch entscheidende Fortschritte bei der Optimierung des Serin- und Cystein-Stoffwechsels gemacht worden, sodass auch einefermentative Herstellung von L-Methionin nunmehr realistisch erscheint. Erste Arbeiten in diese Richtung sind demzufolge kürzlich im Stand der Technik beschrieben worden.

Für die fermentative Herstellung von L-Methionin sind folgende Gene/Allele im Stand der Technik bekannt, deren Einsatz zu einer L-Methionin-Überproduktion führen kann:
- metA-Allele wie beschrieben in einer Anmeldung des gleichen Anmelders vom 11.10.2002 oder in JP2000139471A. Diese metA-Allele kodieren für O-Homoserin-Transsuccinylasen, die einer verminderten Feedback-Hemmung durch L-Methionin unterliegen. Dadurch wird die Bildung von O-Succinylhomoserin weitgehend vom Methionin-Spiegel der Zelle entkoppelt.
- metJ-Deletion wie beschrieben In JP2000139471A
   Das metJ-Gen kodiert für einen zentralen Genregulator des Methioninstoffwechsels und spielt somit eine entscheidende Rolle bei der Expressionskontrolle der Methionin-Biosynthesegene.

Aus dem Stand der Technik Ist ebenfalls naheliegend, dass bekannte Maßnahmen, die eine verbesserte Synthese von L-Serin und L-Cystein gewährleisten, positiven Einfluß auf die Produktion von L-Methionin haben.

Aufgabe der vorliegenden Erfindung ist es, ein Verfahren für die Herstellung von L-Methionin mittels eines Mikroorganismenstammes zur Verfügung zu stellen.

Die Aufgabe wird gelöst durch ein Verfahren bei dem ein Mikroorganismenstamm, der zur fermentativen Herstellung von L-Methionin geeignet ist, in einer Fermentation eingesetzt und L-Methionin aus dem Fermentationsansatz abgetrennt wird, dadurch gekennzeichnet, dass ein Mikroorganismenstamm, verwendet wird, der herstellbar ist aus einem Ausgangsstamm, und der eine gegenüber dem Ausgangsstamm erhöhte Aktivität eines yjeH-Genprodukts oder eines Genprodukts einer funktionellen Allelvariante des yjeH-Gens, die bei einer Analyse mit dem Algorithmus BESTFIT (GCG Wisconsin Package, Genetics Computer Group (GCG) Madison, Wisconsin) eine Sequenzidentität von größer 30 % zu SEQ ID No. 1 aufweist, besitzt, wobei die enzymatische Aktivität des jeweiligen Genproduktes jedoch erhalten bleibt und der Mikroorganismenstamm in einem Fermenter als kontinuierliche Kultur, als batch-Kultur oder vorzugsweise als fed-batch-Kultur angezogen wird.

Eine Erhöhung der Aktivität des yjeH-Genprodukts ist im Sinne der vorliegenden Erfindung auch dann gegeben, wenn durch eine Erhöhung der Genproduktmenge in der Zelle eine erhöhte Gesamtaktivität In der Zelle erreicht wird und somit die spezifische Aktivität des Genprodukts zwar unverändert bleibt, aber die Aktivität des yjeH-Genprodukts pro Zelle erhöht ist

Das yjeH-Gen von Escherichla coll wurde im Rahmen der Genomsequenzierung (Blattner et al. 1997, Science 277:1453-1462) als offener Leserahmen identifiziert und kodiert für ein Protein mit 418 Aminosäuren. Bisher konnte dem yjeH-Gen keine physiologische Funktion zugeordnet werden. Auch eine Datenbankrecherche nach Proteinen mit Sequenzhomologie (FASTA-Algorithmus von GCG Wisconsin Package, Genetics Computer Group (GLG) Madison, Wisconsin) liefert wenig Aufschluß, da signifikante Ähnlichkeiten lediglich zu Proteinen angezeigt werden, deren Funktion ebenfalls unbekannt Ist.

Das yjeH-Gen und das yjeH-Genprodukt (YjeH-Protein) sind durch die Sequenzen SEQ ID No. 1 beziehungsweise SEQ ID No. 2 charakterisiert. Im Rahmen der vorliegenden Erfindung sind als yjeH-Allele vorzugsweise solche Gene aufzufassen, die bei einer Analyse mit dem Algorithmus BESTFIT (GCG Wisconsin Package, Genetics Computer Group (GLG) Madison, Wisconsin) eine Sequenzidentität von größer 53 %, besonders bevorzugt größer 70 % aufweisen, soweit es sich um funktionelle Varianten handelt, die sich durch Deletion, Insertion oder Substitution von Nukleotiden aus der In SEQ ID No. 1 dargestellten Sequenz ableiten, wobei die enzymatische Aktivität des jeweiligen Genprodukts jedoch erhalten bleibt.

Ebenso sind Proteine mit einer Sequenzidentität von größer 30 % (Algorithmus BESTFIT (GCG Wisconsin Package, Genetics Computer Group (GLG) Madison, Wisconsin), vorzugsweise größer 53% als YjeH-homologe Proteine aufzufassen. Besonders bevorzugt ist eine Sequenzidentität von größer 70 %.

Geeingnete Mikroorganismen, die eine gegenüber dem Ausgangsstamm erhöhte Aktivität des yjeH-Genprodukts aufweisen, können mit Standardtechniken der Molekularbiologie erzeugt werden.

Als Ausgangsstämme sind prinzipiell alle Organismen geeignet, die den Biosyntheseweg für L-Methionin aufweisen, rekombinanten Verfahren zugänglich sind und durch Fermentation kuftivierbar sind. Solche Mikroorganismen können Pilze, Hefen oder Bakterien sein. Bevorzugt handelt es sich um Bakterien der phylogenetischen Gruppe der Eubacteria. Besonders bevorzugt sind Mikroorganismen der Familie Enterobacteriaceae und insbesondere der Art Escherichia coli.

Die Erhöhung der Aktivität des yjeH-Genprodukts im Mikroorganismus wird beispielsweise durch eine verstärkte Expression des yjeH-Gens erreicht Dabei kann die Kopienzahl das yjeH-Gens in einem Mikroorganismus erhöht sein und/oder es kann durch geeignete Promotoren die Expression des yjeH-Gens gesteigert sein. Unter verstärkter Expression Ist dabei vorzugsweise zu verstehen, dass das yjeH-Gen mindestens doppelt so stark exprimiert wird wie im Ausgangsstamm.

Die Erhöhung der Kopienzahl des yjeH-Gens In einem Mikroorganismus kann mit dem Fachmann bekannten Methoden vorgenommen werden. So kann zum Beispiel das yjeH-Gen in Plasmid-Vektoren mit mehrfacher Kopienzahl pro Zelle (z.B. pUC19, pBR322, pACYC184 für Escherichia coli) kloniert und in den Mikroorganismus eingebracht werden. Alternativ kann das yjeH-Gen mehrfach ins Chromosom eines Mikroorganismus Integriert werden. Als Integrationsverfahren können die bekannten Systeme mit temperenten Bakteriophagen, integrative Plasmide oder die Integration über homologe Rekombination genutzt werden (z.B. Hamilton et al., 1989, J. Bacteriol. 171: 4617-4622).

Bevorzugt ist die Erhöhung der Kopienzahl durch Klonierung eines yjeH-Gens in Plasmid-Vektoren unter der Kontrolle eines Promotors. Besonders bevorzugt Ist die Erhöhung der Kopienzahl in Escherichia coli durch Klonierung eines yjeH-Gens in einem pACYC-Derivat wie z. B. pACYC184-LH (hinterlegt gemäß Budapester Vertrag bei der Deutschen Sammlung für Mikroorganismen und Zellkulturen, Braunschweig am 18.8.95 unter der Nummer DSM 10172).

Als Kontrollregion für die Expression eines plasmid-kodierten yjeH-Gens kann die natürliche Promotor- und Operatorregion des Gens dienen.

Die verstärkte Expression eines yjeH-Gens kann Jedoch Insbesondere auch mittels anderer Promotoren erfolgen. Entsprechende Promotorsysteme wie beispielsweise in Escherichia coli der konstitutive GAPDH-Promotor des gapA-Gens oder die induzierbaren lac-, tac-, trc-, lambda-, ara oder tet-Promotoren sind dem Fachmann bekannt (Makrides S. C., 1996, Microbiol Rev. 60: 512-538). Solche Konstrukte können In an sich bekannter Weise auf Plasmiden oder chromosomal verwendet werden.

Des weiteren kann eine verstärkte Expression dadurch erreicht werden, dass Translationsstartsignale, wie z. B. die Ribosomenbindestelle oder das Startkodon des Gens, in optimierter Sequenz auf dem jeweiligen Konstrukt vorhanden sind oderdass gemäß der "codon usage" seltene Kodons gegen häufigervorkommende Kodons ausgetauscht werden.

Mikroorganismenstämme mit den genannten Modifikationen werden bevorzugt im erfindungsgemäßen Verfahren eingesetzt.

Die Klonierung eines yjeH-Gens in Plasmid-Vektoren erfolgt beispielsweise durch spezifische Amplifikation mittels der Polymerase-Ketten-Reaktion unter Einsatz von spezifischen Primern, die das komplette yjeH-Gen erfassen, und anschließende Ligation mit Vektor-DNS-Fragmenten.

Als bevorzugte Vektoren für die Klonierung eines yjeH-Gens werden Plasmide verwendet, die bereits Promotoren zur verstärkten Expression enthalten, beispielsweise den konstitutiven GAPDH-Promotor des gapA-Gens von Escherichia coli.

Des weiteren sind Vektoren besonders bevorzugt die bereits ein Gen/Allel enthalten, dessen Einsatz zu einer verminderten Feedback-Hemmung des L-Methionin-Stoffwechsels führt, wie beispielsweise ein mutiertes metA-Allel (beschrieben in der Anmeldung DE-A-10247437). Solche Vektoren ermöglichen die direkte Herstellung von erfindungsgemäßen Mikroorganismenstämmen mit hoher Aminosäure-Überproduktion aus einem beliebigen Mikroorganismenstamm, da ein solches Plasmid auch eine Verminderung der Feedback-Hemmung des Methionin-Stoffwechsels in einem Mikroorganismus bewirkt.

Durch eine gängige Transformationsmethode (z.B. Elektroporation) werden die yjeH-haltigen Plasmide in Mikroorganismen eingebracht und beispielsweise mittels Antibiotika-Resistenz auf plasmid-tragende Klone selektiert.

Besonders bevorzugt als Stämme für die Transformation mit oben genannten Plasmiden sind solche, die chromosomal bereits Allele aufweisen, die ebenfalls eine L-Methionin-Produktion begünstigen können, wie beispielsweise
- eine matJ-Deletion(wie In JP2000139471A beschrieben) oder
- Allele, die eine verbesserte Serin-Bereitstellung bewirken, wie feedbackresistente serA-Varianten (wie beispielsweise In EP0620853B1 oder EP0931833A2 beschrieben)
- oder Gene, die eine verbesserte Cystein-Bereitstellung bewirken, wie feedbackresistente cysE-Varianten (wie beispielsweise in WO 97/15673 beschrieben)

Die Produktion von L-Methionin mit Hilfe eines derartigen Mikroorganismenstammes erfolgt in einem Fermenter nach an und für sich bekannten Verfahren.

Die Erfindung betrifft somit auch ein Verfahren zur Herstellung von L-Methionin, welches dadurch gekennzeichnet ist, dass ein erfindungsgemäßer Mikroorganismenstamm in einer Fermentation eingesetzt wird und das produzierte L-Methionin aus dem Fermentationsansatz abgetrennt wird.

Die Anzucht des Mikroorganismenstammes im Fermenter erfolgt als kontinuierliche Kultur, als batch-Kultur oder vorzugsweise als fed-batch-Kultur. Besonders bevorzugt wird eine C-Quelle während der Fermentation kontinuierlich zudosiert.

Als C-Quelle dienen vorzugsweise Zucker, Zuckeralkohole oder organische Säuren. Besonders bevorzugt werden im erfindungsgemäßen Verfahren als C-Quellen Glukose, Laktose oder Glycerin eingesetzt

Bevorzugt Ist die Dosierung der C-Quelle in einer Form, die gewährleistet, dass der Gehalt an C-Quelle Im Fermenter während der Fermentation In einem Bereich von 0,1 - 50 g/l gehalten wird. Besonders bevorzugt Ist ein Bereich von 0,5 -10 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert

Als weitere Medienzusätze können Salze der Elemente Phosphor, Chlor, Natrium, Magnesium, Stickstoff, Kalium, Calcium, Eisen und In Spuren (d.h. in µM Konzentrationen) Salze der Elemente Molybdän, Bor, Kobalt, Mangan, Zink und Nickel zugesetzt werden.

Des weiteren können organische Säuren (z.B. Acetat, Citrat), Aminosäuren (z.B. Leucin) und Vitamine (z.B. B1, B12) dem Medium zugesetzt werden.

Als komplexe Nährstoffquellen können z.B. Hefeextrakt, Maisquellwasser, Sojamehl oder Malzextrakt zum Einsatz kommen.

Die inkubationstemperatur für mesophile Mikroorganismen beträgt vorzugsweise 15 - 45 °C, besonders bevorzugt 30 - 37 °C.

Die Fermentation wird vorzugsweise unter aeroben Wachstumsbedingungen durchgeführt. Der Sauerstoffeintrag in den Fermenter erfolgt mit Druckluft oder mit reinem Sauerstoff.

Der pH-Wert des Fermentationsmediums liegt während der Fermentation bevorzugt im pH-Bereich von 5,0 bis 8,5, besonders bevorzugt ist ein pH-Wert von 7,0.

Für die Herstellung von L-Methionin kann während der Fermentation eine Schwefelquelle zugefüttert werden. Bevorzugt kommen dabei Sulfate oder Thiosulfate zum Einsatz.

Mikroorganismen, die nach dem beschriebenen Verfahren fermentiert werden, sezernieren in einem Batch- oder Fedbatch-Prozess nach einer Anwachsphase in einem Zeitraum von 10 bis 150 Stunden L-Methionin in das Kulturmedium.

Das produzierte L-Methionin kann mit gängigen Maßnahmen zur Aminosäureisolierung aus Fermenterbrühen gewonnen werden (z.B. lonentauscherverfahren, Kristallisation etc.).

Die folgenden Beispiele dienen der weiteren Erläuterung der Erfindung. Als ein Bakterienstamm mit einem erfindungsgemäßen Plasmid mityjeH-Gen, derzurerfindungsgemäßen Herstellung von L-Methionin geeignet ist, wurde der:Stamm W3110ΔJ/pKP450 bei der DSMZ (Deutsche Sammlung für Mikroorganismen und Zellkulturen GmbH, D-38142 Braunschweig) unter der Nummer DSM 15421 gemäß Budapester Vertrag hinterlegt.

### Beispiel 1: Klonierung des Basisvektors pKP228

Um das yjeH-Gene unter Kontrolle eines konstitutiven Promotors zu setzen, wurde zunächst ein Basisvektor mit dem konstitutiven GAPDH-Promotor des gapA-Gens für die Glycerinaldehyd-3-dehydrogenase von Escherichia coli konstruiert. Hierfür wurde eine Polymerase-Kettenreaktion mit den Primern und chromosomaler DNS des E. coli Stamms W3110 (ATCC27325) durchgeführt. Das resultierende DNS-Fragment wurde mit Hilfe einer Agarose-Gelelektrophorese gereinigt und anschließend isoliert (Qiaquick Gel Extraction Kit, Qiagen, Hilden, D) Danach wurde das Fragment mit den Restriktionsenzymen Pacl und Miui behandelt und in den ebenfalls Pacl/Mlul geschnittenen Vektor pACYC184-LH (hinterlegt gemäß Budapester Vertrag bei der Deutschen Sammlung für Mikroorganismen und Zellkulturen, Braunschweig am 18.8.95 unter der Nummer DSM 10172) kloniert. Das neue Konstrukt wurde mit der Bezeichnung pKP228 benannt.

### Beispiel 2: Klonierung des yjeH-Gens

Das yjeH-Gen aus Escherichia coli Stamm W3110 wurde mit Hilfe der Polymerase-Kettenreaktion amplifiziert. Als spezifische Primer dienten die Oligonukleotide
**yjeH-fw:** (**SEQ**. **ID**. **NO: 5)**
**5'- ATT GCT GGT TTG CTG CTT-3'**
und
**yjeH**-**rev:** (**SEQ**. **ID**. **NO: 6)**
**5**'- **AGC ACA AAA TCG GGT GAA-3'**
sowie chromosomale DNS des E. coli Stammes W3110 (ATCC27325) als Matrize. Das resultierende DNS-Fragment wurde durch eine Agarose-Gelelektrophorese-gereinigt und isoliert (Qiaquick Gel Extraction Kit, Qiagen, Hilden, Deutschland). Die Klonierung erfolgte durch blunt end-Ligation mit einem Bglllgeschnittenen Vektor pKP228, dessen 5'-überhängende Enden mit Klenow-Enzym aufgefüllt wurden. Durch das angegebene Vorgehen wird das yjeH-Gen so hinter dem GAPDH-Promotor plaziert, dass die Transkription von dort aus initiiert werden kann. Der resultierende Vektor trägt die Bezeichnung pKP450.

### Beispiel 3: Kombination von yjeH-Gen mit einem feedbackresistenten metA-Allel

Ein in der Patentanmeldung DE-A-10247437 des gleichen Anmelders vom 11.10.2002 beschriebenes metA-Allel, das für eine feedbackresistente O-Homoserin-Transsuccinylase kodiert, wurde durch Polymerase-Ketten-Reaktion mit dem Template pKP446 (ebenfalls beschrieben in der Patentanmeldung DE-A-10247437) und den Primern amplifiziert. Dabei wurden terminale Schnittstellen für die Restriktionsedonukleasen Ncol und Sacl generiert. Das erhaltene DNS-Fragment wurde mit eben diesen Endonukleasen verdaut, gereinigt und in den Ncol/Sacl-geschnittenen Vektor pKP450 kloniert. Das resultierende Plasmid erhielt die Bezeichnung pKP451.
Um ein Kontrollplasmid mit metA-Allel, aber ohne yjeH-Gen, herzustellen, wurde das yjeH-Gen aus pKP451 deletiert. Dazu wurde pKP451 mit Ecl136II und Pacl geschnitten, die überhängenden Enden mit Klenow-Enzym abgedaut und der Vektor religiert. Das so erhaltene Plasmid trägt die Bezeichnung pKP446AC.

### Beispiel 4: Erzeugung einer chromosomalen metJ-Mutation

Die Gene metJ/B wurden durch Polymerase-Ketten-Reaktion mit den Primer
**metJ-fw: (SEQ. ID. NO: 9)**
**5'-GAT CGC GGC CGC TGC AAC GCG GCA TCA TTA AAT TCG A**-**3**'
und
**metJ-rev: (SEQ. ID. NO: 10)**
**5'-GAT CGC GGC CGC AGT TTC AAC CAG TTA ATC AAC TGG-3'**
und chromosomaler DNS aus Escherichia coli W3110 (ATCC27325) amplifiziert. Das 3,73 Kilobasen enthaltende Fragment wurde gereinigt, mit der Restriktionsendonuklease Notl verdaut und in den Notl-geschnittenen Vektor pACYC184-LH (siehe Beispiel 1) kloniert. Danach wurde in das metJ-Gen an der internen AfIIII-Schnittstelle eine Kanamycin-Resistenzkassette inseriert. Hierfür wurde erst mit AfIIII verdaut und mit Klenow-Enzym glatte Enden erzeugt. Die Kanamycin-Kassette wurde wiederum durch Pvull-Restriktion aus dem Vektor pUK4K (Amersham Pharmacia Biotech, Freiburg, D) gewonnen und durch Ligation in das metJ-Gen inseriert. Aus dem so hergestellten Vektor pKP440 wurde nun die metJ::kan-Kassette durch Notl-Restriktion als lineares Fragment gewonnen und nach der Methode von Winans et al. (J. Bacteriol. 1985, 161:1219-1221) in den recBC/sbcB-Stamm JC7623 (E.coli Genetic Stock Center CGSC5188) chromosomal integriert. Als letzter Schritt wurde schließlich die metJ::kan-Mutation durch P1-Transduktion (Miller, 1972, Cold Spring Harbour Laboratory, New York, S. 201-205) in den Wildtypstamm W3110 (ATCC27325) transduziert und somit der Stamm W3110ΔJ erzeugt.
Nach der Verifizierung der metJ::kan-lnsertion wurde der Stamm W3110ΔJ jeweils mit den yjeH-tragenden Plasmiden bzw. den Kontrollplasmiden transformiert und entsprechende Transformanten mit Tetracyclin selektiert.

### Beispiel 5: Vorkultur von Produktionsstämmen für die Fermentation

Als Vorkultur für die Fermentation wurden 20 ml LB-Medium (10 g/l Trypton, 5 g/l Hefeextrakt, 10 g/l NaCl), das zusätzlich 15 mg/l Tetracyclin enthielt, mit den Produktionsstämmen beimpft und bei 30°C und 150 rpm in einem Schüttler inkubiert. Nach sieben Stunden wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ × 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 15 mg/l Tetracyclin supplementiert wurde, überführt. Die weitere Inkubation erfolgte bei 30 °C für 17 Stunden bei 150 rpm.

### Beispiel 6: Fermentative Herstellung von L-Methionin

Als Fermenter diente ein Biostat B-Gerät der Firma Braun Biotech (Melsungen, D) mit einem maximalen Kulturvolumen von 2 1. Mit der in Beispiel 5 beschriebenen Vorkultur (optische Dichte bei 600 nm von ca. 3) wurde der Fermenter mit 900 ml SM1-Medium, das mit 15 g/l Glukose, 10 g/l Trypton, 5 g/l Hefeextrakt, 3 g/l Na₂S₂O₃x5H₂0, 0,5 mg/l Vitamin B₁, 30 mg/l Vitamin B₁₂ und 15 mg/l Tetracyclin supplementiert wurde, beimpft. Während der Fermentation wurde eine Temperatur von 32 °C eingestellt und der pH-Wert durch Zudosierung von 25 % Ammoniak bei einem Wert von 7,0 konstant gehalten. Die Kultur wurde mit entkeimter Druckluft bei 5 vol/vol/min begast und mit einer Rührerdrehzahl von 400 rpm gerührt. Nach Absinken der Sauerstoffsättigung auf einen Wert von 50 % wurde die Drehzahl über ein Kontrollgerät bis zu einem Wert von 1500 rpm erhöht, um 50 % Sauerstoffsättigung zu erhalten (Bestimmt mit einer pO₂₋Sonde, kalibriert auf 100% Sättigung bei 900 rpm). Sobald der Glukose-Gehalt im Fermenter von anfänglich 15 g/l auf ca. 5-10 g/l abgesunken war, erfolgte eine Zudosierung einer 56 % Glukose-Lösung. Die Fütterung erfolgte mit einer Flußrate von 6-12 ml/h, wobei die Glukosekonzentration im Fermenter zwischen 0,5 - 10 g/l konstant gehalten wurde. Die Glukose-Bestimmung wurde mit dem Glukoseanalysator der Firma YSI (Yellow Springs, Ohio, USA) durchgeführt. Die Fermentationsdauer betrug 48 Stunden. Nach dieser Zeit wurden Proben entnommen und die Zellen durch Zentrifugation vom Kulturmedium abgetrennt. Die resultierenden Kulturüberstände wurden durch reversed phase HPLC an einer LUNA 5 µ C18(2)-Säule (Phenomenex, Aschaffenburg, Deutschland) bei einer Flußrate von 0,5 ml/min analysiert. Als Eluent diente verdünnte Phosphorsäure (0,1 ml konz. Phosphorsäure / 1). Die Tabelle 1 zeigt die erzielten Gehalte an L-Methionin im Kulturüberstand.

**Tabelle 1:**

| **Stamm** | **Genotyp (Plasmid)** | **L-Methionin [g/l]** |
|---|---|---|
| W3110ΔJ / pKP228 | - | < 0,1 g/l |
| W3110ΔJ / pKP450 | yjeH | 0,8 g/l |
| W3110ΔJ / pKP451 | metA^{fbr} yjeH | 4,8 g/l |
| W3110ΔJ / pKP446AC | metA^{fbr} | 0,9 g/l |
| fbr feedbackresistent | | |

## Patentansprüche

1. Verfahren zur Herstellung von L-Methionin bei der ein Mikroorganismenstamm, der zur fermentativen Herstellung von L-Methionin geeignet ist, in einer Fermentation eingesetzt und L-Methionin aus dem Fermentationsansatz abgetrennt wird, **dadurch gekennzeichnet, dass** ein Mikroorganismenstamm, verwendet wird, der herstellbar ist aus einem Ausgangsstamm, und der eine gegenüber dem Ausgangsstamm erhöhte Aktivität eines yjeH-Genprodukts oder eines Genprodukts einer funktionellen Allelvariante des yjeH-Gens, die bei einer Analyse mit dem Algorithmus BESTFIT (GCG Wisconsin Package, Genetics Computer Group (GCG) Madison, Wisconsin) eine Sequenzidentität von größer 30 % zu SEQ ID No. 1 aufweist, besitzt, wobei die enzymatische Aktivität des jeweiligen Genproduktes jedoch erhalten bleibt und der Mikroorganismenstamm in einem Fermenter als kontinuierliche Kultur, als batch-Kultur oder vorzugsweise als fed-batch-Kultur angezogen wird.

2. Verfahren gemäß Anspruch 1 **dadurch gekennzeichnet, dass** das Genprodukt einer funktionellen Allelvariante des yjeH-Gens, bei einer Analyse mit dem Algorithmus BESTFIT (GCG Wisconsin Package, Genetics Computer Group (GCG) Madison, Wisconsin) eine Sequenzidentität von größer 53 % vorzugsweise von größer 70% zu SEQ ID No. 1 aufweist.

3. Verfahren gemäß Anspruch 1 oder 2 **dadurch gekennzeichnet, dass** es sich bei dem Mikroorganismenstamm um einen Stamm der Familie Enterobacteriaceae, besonders bevorzugt der Art Escherichia coli handelt.

4. Verfahren gemäß Anspruch 4 **dadurch gekennzeichnet, dass** es sich um einen Escherichia coli Stamm mit einer metJ Deletion handelt.

5. Verfahren gemäß Anspruch 1, 2, 3 oder 4, **dadurch gekennzeichnet, dass** die Kopienzahl des yjeH-Gens in dem Mikroorganismus erhöht ist oder die Expression des yjeH-Gens durch Einsatz geeigneter Promotoren oder Translationssignale gesteigert ist.

6. Verfahren nach einem der Ansprüche 1 bis 5, **dadurch gekennzeichnet, dass** die gegenüber dem Ausgangsstamm erhöhte Aktivität eines yjeH-Genprodukts durch eine mindestens doppelt so starke Expression des yjeH-Gens wie im Ausgangsstamm erreicht wird.

7. Verfahren gemäß Anspruch 5, **dadurch gekennzeichnet, dass** der Promotor ausgewählt ist aus der Gruppe konstitutiver GAPDH-Promotor des gapA-Gens, induzierbarer lac-, tac-, trc-, lambda-, ara und tet-Promotor.

8. Verfahren nach einem der Ansprüche 1 bis 7 **dadurch gekennzeichnet, dass** es sich bei dem Mikroorganismenstamm um einen Escherichia coli Stamm handelt, bei dem die erhöhte Aktivität eines yjeH-Genprodukts auf der Erhöhung der Kopienzahl des yjeH-Gens in einem pACYC-Derivat beruht.

9. Verfahren nach einem der Ansprüche 1 bis 8 **dadurch gekennzeichnet, dass** eine C-Quelle während der Fermentation kontinuierlich zudosiert wird.

10. Verfahren gemäß Anspruch 9, **dadurch gekennzeichnet, dass** als C-Quelle Zucker, Zuckeralkohole oder organische Säuren dienen.

11. Verfahren nach Anspruch 9 oder 10, **dadurch gekennzeichnet, dass** die Dosierung der C-Quelle in einer Form erfolgt, die gewährleistet, dass der Gehalt an C-Quelle im Fermenter während der Fermentation in einem Bereich von 0,1 - 50 g/l, besonders bevorzugt ist ein Bereich von 0,5 - 10 g/l, gehalten wird.

12. Verfahren nach einem der Ansprüche 9 bis 11, **dadurch gekennzeichnet, dass** als N-Quelle Ammoniak, Ammoniumsalze oder Proteinhydrolysate verwendet werden.

13. Verfahren nach einem der Ansprüche 1 bis 12, **dadurch gekennzeichnet, dass** die Fermentation unter aeroben Wachstumsbedingungen erfolgt.

## Claims

1. Method for preparing L-methionine, in which a microorganism strain suitable for fermentative production of L-methionine is used in a fermentation and L-methionine is removed from the fermentation mixture, **characterized in that** a microorganism strain is used which is preparable from a starting strain, and which has an increased activity, compared to the said starting strain, of a yjeH gene product or of a gene product of a functional allele variant of the yjeH gene whose sequences are more than 30% identical to SEQ ID No. 1 in an analysis using the BESTFIT algorithm (GCG Wisconsin Package, Genetics Computer Group (GCG) Madison, Wisconsin), with the enzymatic activity of the respective gene product being retained, however, and the microorganism strain being grown as continuous culture, as batch culture or preferably as fed-batch culture in a fermenter.

2. Method according to Claim 1, **characterized in that** the sequence of the gene product of a functional allele variant of the yjeH gene, in an analysis using the BESTFIT algorithm (GCG Wisconsin Package, Genetics Computer Group (GCG) Madison, Wisconsin), is more than 53%, preferably more than 70%, identical to SEQ ID No. 1.

3. Method according to Claim 1 or 2, **characterized in that** the microorganism strain is a strain of the Enterobacteriaceae family, particularly preferably of the species Escherichia coli.

4. Method according to Claim 4, **characterized in that** the Escherichia coli strain is one with a metJ deletion.

5. Method strain according to Claim 1, 2, 3 or 4, **characterized in that** the copy number of the yjeH gene in the microorganism is increased or expression of the said yjeH gene is increased by using suitable promoters or translation signals.

6. Method of any of Claims 1 to 5, **characterized in that** the increased activity, compared to the starting strain, of a yjeH gene product is achieved by at least twice as strong an expression of the yjeH gene as in the said starting strain.

7. Method according to Claim 5, **characterized in that** the promoter is selected from the group consisting of constitutive GAPDH promoter of the gapA gene, inducible lac, tac, trc, lambda, ara and tet-promoters.

8. Method according to any of Claims 1 to 7, **characterized in that** the microorganism strain is an Escherichia coli strain in which the increased activity of a yjeH gene product is based on increasing the copy number of the yjeH gene in a pACYC derivative.

9. Method according to any of Claims 1 to 8, **characterized in that** a carbon source is continuously metered in during fermentation.

10. Method according to Claim 9, **characterized in that** the carbon source used is sugar, sugar alcohols or organic acids.

11. Method according to Claim 9 or 10, **characterized in that** the carbon source is metered in so as to ensure that the carbon source content in the fermenter is maintained within a range from 0.1-50 g/l during fermentation, with range from 0.5-10 g/l being particularly preferred.

12. Method according to any of Claims 9 to 11, **characterized in that** the nitrogen source used is ammonia, ammonium salts or protein hydrolysates.

13. Method according to any of Claims 1 to 12, **characterized in that** the fermentation is carried out under aerobic growth conditions.

## Revendications

1. Procédé de fabrication de L-méthionine, dans laquelle une souche de microorganisme, qui convient à la fabrication fermentative de L-méthionine, est utilisée dans une fermentation et la L-méthionine est séparée de la fraction de fermentation, **caractérisé en ce qu'**on utilise une souche de microorganisme que l'on peut fabriquer à partir d'une souche de départ, et qui présente une activité élevée, par rapport à la souche de départ, d'un produit génique du gène yjeH ou d'un produit génique d'une variante d'allèle fonctionnelle du gène yjeH, laquelle présente, dans le cas d'une analyse utilisant l'algorithme BESTFIT (GCG Wisconsin Package, Genetics Computer Group (GCG) Madison, Wisconsin), une identité de séquence de plus de 30% par rapport à la SEQ ID n° 1, l'activité enzymatique du produit génique respectif étant cependant maintenue et la souche de microorganisme étant cultivée dans un fermenteur sous la forme d'une culture en continu, d'une culture en discontinu ou de préférence d'une culture semi-discontinue.

2. Procédé selon la revendication 1, **caractérisé en ce que** le produit génique d'une variante d'allèle fonctionnelle du gène yjeH présente, dans le cas d'une analyse utilisant l'algorithme BESTFIT (GCG Wisconsin Package, Genetics Computer Group (GCG) Madison, Wisconsin), une identité de séquence de plus de 53%, de préférence de plus de 70%, par rapport à la SEQ ID n° 1.

3. Procédé selon la revendication 1 ou 2, **caractérisé en ce que** la souche de microorganisme est une souche de la famille Enterobacteriaceae, particulièrement préférablement du type Escherichia coli.

4. Procédé selon la revendication 4, **caractérisé en ce qu'**il s'agit d'une souche de Escherichia coli avec une délétion metJ.

5. Procédé selon la revendication 1, 2, 3 ou 4, **caractérisé en ce que** le nombre de copies du gène yjeH dans le microorganisme est augmenté ou **en ce que** l'expression du gène yjeH est accrue en utilisant des promoteurs ou des signaux de traduction appropriés.

6. Procédé selon l'une quelconque des revendications 1 à 5, **caractérisé en ce que** l'activité d'un produit génique yjeH élevée par rapport à la souche de départ est atteinte par une expression du gène yjeH au moins deux fois plus forte que dans la souche de départ.

7. Procédé selon la revendication 5, **caractérisé en ce que** le promoteur est choisi dans le groupe du promoteur constitutif GAPDH du gène gapA, des promoteurs inductibles lac, tac, trc, lambda, ara et tet.

8. Procédé selon l'une quelconque des revendications 1 à 7, **caractérisé en ce que** la souche de microorganisme est une souche Escherichia coli, dans laquelle l'activité plus élevée d'un produit génique du gène yjeH repose sur l'augmentation du nombre de copies du gène yjeH dans un dérivé de pACYC.

9. Procédé selon l'une quelconque des revendications 1 à 8, **caractérisé en ce que** l'on ajoute en dosant en continu une source de carbone pendant la fermentation.

10. Procédé selon la revendication 9, **caractérisé en ce que** des sucres, des alcools de sucres ou des acides organiques servent de source de carbone.

11. Procédé selon la revendication 9 ou 10, **caractérisé en ce que** le dosage de la source de carbone s'effectue sous une forme qui garantisse que la teneur en source de carbone dans le fermenteur soit maintenue dans une plage de 0,1 à 50 g/l lors de la fermentation, une plage de 0,5 à 10 g/l étant particulièrement préférée.

12. Procédé selon l'une quelconque des revendications 9 à 11, **caractérisé en ce que** l'on utilise comme source d'azote de l'ammoniac, des sels d'ammonium ou des hydrolysats de protéines.

13. Procédé selon l'une quelconque des revendications 1 à 12, **caractérisé en ce que** la fermentation s'effectue dans des conditions de croissance aérobies.
